# EUROPEAN PATENT APPLICATION

(11) **EP 2 859 915 A1**
(43) Date of publication of application: **15.04.2015**
(21) Application number: 14460065.7
(22) Date of filing: 09.10.2014
(51) Int. Cl.: A61N 5/06, A61M 1/32, A61M 1/36, A61M 1/16

(54) **Device for blood photobiomodulation during the extracorporeal circulation**

(30) Priority: 10.10.2013 PL 40560213
(71) Applicant: Wojewodzki Szpital Specjalistyczny we Wroclawiu, 51-124 Wroclaw (PL)
(72) Inventor: Walski, Tomasz, 46-040 Ozimek (PL); Komorowska, Malgorzata, 51-659 Wroclaw (PL); Grzeszczuk-Kuc, Karolina, 53-412 Wroclaw (PL); Galecka, Katarzyna, 50-321 Wroclaw (PL); Oleszko, Adam, 45-286 Opole (PL)
(74) Representative: Winohradnik, J. Halina

(57) **Abstract**

The present invention is a device, for the blood photomodulation during the extracorporeal circulation, used in the cardiovascular surgery, transplantation and the heart or respiratory failure treatment.

The device comprises at least one R/NIR emitter (1), oxygenator (2) and heat exchanger (4), wherein the R/NIR emitter (1) is positioned in relation to the oxygenator (2) and heat exchanger (4) in the way enabling the irradiation of flowing blood. The R/NIR emitter comprises at least one source of electromagnetic waves emitting radiation with one or more wavelengths, in the range of 700 to 1500 nm. Furthermore, the distribution of EM wave sources in the space enable to provide uniform irradiation of the flowing blood.

In an another embodiment the device comprises the blood filter (8) or pump (9) assisting the blood flow.

In a preferred embodiment according to the present invention, the oxygenator is integrated with the heat exchanger or the oxygenator is integrated with the heat exchanger and blood filter or the oxygenator is integrated with the heat exchanger, blood filter and the pump.

The emitter consist the EM waves source which is selected from the following group: LED, incandescent, fluorescent, sodium, halogen, laser lamp or other device that emits light in a pre-defined range of wavelengths or emitting light of a different spectral range which can be converted to light in the range of 700 to 1500 nm.

## Description

The present invention is a device, for the blood photomodulation during the extracorporeal circulation, used in the cardiovascular surgery, transplantation and the heart or respiratory failure treatment.

During the surgery, it is sometimes indispensable to use the heart-lung machine, especially necessary to carry out open-heart procedures. Devices, applied for this reason, includes: the patient's blood pump which drives the extracorporeal blood circulation, drainage supplying blood to the artificial lung responsible for the gas exchange (oxygenators), a heat exchanger to maintain the temperature of blood at a given level, the cardiotomy container, hemoconcentrator, a blood filter and a cannulas connecting the machine with the patient's circulatory system.

The technique of ECMO (ang. Extracorporeal Membrane Oxygenation) is based on the artificial heart-lung and is used in the treatment of reversible acute respiratory failure and acute heart failure, in intensive care units. In the suitable modified variants cardiopulmonary bypass can support the patient's respiratory system (veno-venous ECMO) or both respiratory and circulation system (veno-arterial ECMO). Also, the systems mainly for supporting the gas exchange with particular regard to the carbon dioxide removal (Extracorporeal Lung Assist, ECLA) are applied, which do not use a mechanical pump, and the blood flow is caused by the difference in blood pressure between the artery and the vein of patient. Extracorporeal oxygenation systems are indicated for use in cases of acute respiratory or circulatory failure, resistant to conventional methods, when the underlying disease process is reversible, possible to cure and there are no absolute contraindications [see for example, *Grzybowski, A. (2003) et al. Pozaustrojowe utlenowanie krwi (ECMO),* Wydawnictwo Ślask].

An oxygenator as an element of the extracorporeal circulation of blood used for its oxygenation is known from the U.S. Patents No. US2934067 and No. US5043140. The Patent No. US8133195 describes the oxygenator integrated with heat exchanger and blood filter, which allows for the lower dilution of the patient's blood, to assist the regeneration process after the surgical procedure, and also allows to reduce in the number of red blood cells concentrates transfusions and medications needed for surgery.

Various devices for modification of extracorporeal circulation with physicals and chemical factors are known and used. Patent No. US2309124 describes an apparatus for the extracorporeal irradiation of blood, to profit from the antibacterial properties of UV radiation. Device disclosed in the patent EP1689450 (container for extracorporeal blood irradiation with UV), allows for the implementation of the method, involving the dislocation of patient's blood with the natural blood pressure into the container of oblong shape with the opening where a cassette that allows the irradiation is placed.

Known from Patent US4692138 pump block used as interface of irradiation chamber with a roller pump is connected to the extracorporeal circuit to take the patient's blood, separation of the blood cellular components and dosing of photosensitizers to the selected group of cells. Irradiation of leukocytes results in the activation of the photosensitizer and in consequence - immunosuppression. The blood is returned to the patient, in organism of whom blood is purified from over-activated cells. A very similar solution, also with UV radiation is described in U.S. Patent No. US4612007.

Patents Nos US4321918 and US4576143 describes an apparatus, process and the method of extracorporeal irradiation of the whole blood with X-rays to alter the limphocyte function and to modify the immune response in humans suffering from autoimmune diseases and to inhibit the immune response to the transplanted organs. X-ray is applied through coiled tubing of the extracorporeal circuit. The flow is driven by a peristaltic pump.

Known from the patent Nos. US4950225 and US5104373 apparatus and method for hyperthermic, extracorporeal treatment of blood-borne diseases, such as, for example the HIV-1 infection of T cells, includes three modules: a) hyperthermic, b) inducing a mechanical stress, c) irradiating. Each of them may be used alone or in combination with other modules in any order and combination. The patient's blood taken with a single-channel catheter is fractionated with use of the apheresis apparatus on leukocytes, plasma and erythrocytes. Leukocytes (particularly T cells) are subjected to the irradiation with the X-ray and/or UV and or laser light within the spectrum of visible and/or visible light. The irradiated cells are then combined with the previously separated blood fractions or the fluid filling the device and returned to the patient. The apparatus further includes an oxygenation system used to stabilize the pH of the circulating cell suspension at 7.2 ± 0.1.

Hemodialysis apparatus described in U.S. Patent No. US7527737, consists of the dialyzer, dialysis fluid, the blood line and sources of the visible and NIR radiation, which are distributed equally and annularly around the bloodline.

Patent No. US8535361 covers the method and the portable system for non-invasive therapy consisting of treating the blood with the radiation within the range of UV-VIS-IR in order to reduce the number of the erythrocyte aggregates and thus lower the blood viscosity in the patient's nostril. Nasal applicator of electromagnetic radiation emitted by the laser is described in Patent Application No. EP2179767.

From the patent application under the PCT WO1999016308 the device and the method of blood exposure to sunlight or an appropriate full spectrum radiation is known. The device includes a base to which the rotating stand for biological samples is connected. The base consists the knob forcing the rotation of the stand. A sample of the whole blood or blood fraction placed in the transfusion container is exposed to electromagnetic radiation falling on the rotating sample. Wherein the radiation is of solar origin or it is generated by a fluorescent light bulb. Lamps are fixed to the frame over the transfusion container. To expose all the cells equally it is necessary to take some additional steps related to the control of rotation. The method of irradiation of blood involves taking a patient's blood to the transfusion container, securing the product, placing it in a rotating stand of the device, exposure to sunlight or full spectrum radiation, blood reinfusion to the patient. The process does not involve additional anticoagulants, assuming that the applied electromagnetic radiation acts as anticoagulant. The described method of treatment is suitable for use in the treatment of infections, the diseases of the cardiovascular system and the inflammatory diseases basing mainly on the properties of the solar light.

Protective photochemical effect of laser He-Ne radiation during extracorporeal circulation on extracorporeal circulation model was described by Itoh T., Murakami H., Orihashi K., Sueda T., Kusumoto Y., Kakehashi M., Matsuura Y. Low power laser protects human erythrocytes In an In vitro model of artificial heart-lung machines. Artif Organs. 2000; 24(11):870-3*.* Circulating suspension of erythrocytes in the heart-lung system was exposed to laser radiation with a wavelength of 632 nm and output power of 8.5 mW. The laser beam was focused on the chamber for the saturation measurement in the perpendicular way to the direction of the suspension flow. During the four-hour experiment a decrease in intracellular ATP and an increase in hemolysis was observed. Radiation caused a reduction of the destructive impact of extracorporeal circulation on the erythrocytes. The Scanning Electron Microscopy analysis of oxygenator membranes revealed a significantly higher number of discocytes in the sample than in the control (respectively 45% and 20%).

A few among the known and used systems for a blood processing are designed for extracorporeal circulation. Most of the them is based on collecting a blood sample from the organism, detachment of the sample from the sampling system, modification of the sample with the radiation and finally - the reinfusion.

It is thus the main object of the present invention is the novel device for blood photobiomodulation with R/NIR radiation, composed of R/NIR emitter irradiating the blood flowing through the oxygenator during the extracorporeal circulation. The device comprises at least one R/NIR emitter, oxygenator and heat exchanger. Wherein the R/NIR emitter is positioned in relation to the oxygenator and heat exchanger in the way enabling the irradiation of blood flowing through the oxygenator or is an integral part.

In a preferred embodiment according to the present invention, the device includes at least one blood filter.

In a preferred embodiment according to the present invention, the device comprises at least one pump, preferably a roller pump or centrifugal pump for assisting blood flow.

In a preferred embodiment according to the present invention, the oxygenator is integrated with the heat exchanger, preferably in a common housing to form a structural unit convenient to use as a component of the extracorporeal circuit.

Furthermore, in the embodiment of the device, the oxygenator is integrated with a heat exchanger and blood filter, optionally in a common housing to form a structural unit of the extracorporeal circuit.

In another embodiment of the device, the oxygenator is integrated with a heat exchanger, a blood filter and pump for assisting blood flow.

The emitter of the R/NIR irradiator, comprises at least one electromagnetic wave source, emitting radiation with one or more wavelengths, in the range of 700 to 1500 nm. The EM wave source is selected from the following group: LED, incandescent, fluorescent, sodium, halogen, laser lamp or other device that emits light in a pre-defined range of wavelengths or emitting light of a different spectral range which can be converted to light in the range of 700 to 1500 nm.

The device has means for attaching the emitter in the vicinity of the blood filter, heat exchanger, a blood flow assisting pump or blood transporting drain for the additional irradiation in the extracorporeal circulation system.

Furthermore, the device which comprising more than one source of EM waves, the distribution of wave sources in the space enables to provide uniform irradiation of the flowing blood. Preferably, the R/NIR emitters compose an integral part of the oxygenator or heat exchanger or a blood filter or drain for direct irradiation of blood flowing through them.

In a preferred embodiment according to the present invention, the device is provided with a fiber optic system, transmitting the R/NIR radiation directly from emitter to the blood flowing through the oxygenator or heat exchanger or a filter or pump or blood transporting drain to provide direct irradiation of blood.

In the device, the irradiance which reaches the blood is in the range of 0.5 mW/cm² to 10000 mW/cm².

In an another embodiment according to the present invention, the device is connected to the cardiotomy container or hemofilter with transporting blood drain.

The emitters may be attached to a single or multiple frames to the oxygenator module, cardiotomy container holder, an independent stand, or other holder retaining them in the best position to allow the delivery of the R/NIR radiation to the blood.

An advantage of the device is providing the exposure of blood flowing through oxygenator on R/NIR radiation, during extracorporeal circulation. The device allows to eliminate the need of collection the blood from the organism, detachment of the blood samples from the sampling system, the modification of the sample with R/NIR radiation, and then the reinfusion. An important advantage is that at least one emitter may be in direct contact with blood, in the embodiment, in which it is an integral part of the oxygenator or heat exchanger or a blood filter, or drain.

The advantage of the device is the ability to supply the radiation beam to blood flowing not only through the oxygenator, but also other extracorporeal circulatory system locations, such as a heat exchanger and blood filter and blood transporting drain on the surface, or directly by a fiber optic system.

Another advantage of the device is the possibility of selection of sources emitting the radiation of different power or different wavelengths, depending on the needs. Furthermore, a beneficial feature is the ability to adjust the power of R/NIR radiation in a continuous or stepper way and also that the R/NIR radiation can be emitted as continuous or pulsing beam. The device according to the invention solves the problem of side effects arising from the use of extracorporeal circulation.

For a better understanding of the invention, as well as features and advantages thereof, reference is made to the accompanying drawings wherein:
- Fig. 1: - presents embodiment of the device for extracorporeal blood exposure to R/NIR radiation
- Fig. 2: - presents embodiment of the device, comprising the oxygenator module integrated with the heat exchanger,
- Fig. 3: - presents embodiment of the device comprising a blood filter.
- Fig. 4: - presents embodiment of the device, comprising the blood filter and the oxygenator unit integrated with a heat exchanger,
- Fig. 5: - presents embodiment of the device, comprising the oxygenator unit integrated with a heat exchanger and a blood filter,
- Fig. 6: - presents embodiment of the device, comprising the pump and the oxygenator unit integrated with a heat exchanger and blood filter,
- Fig. 7: - presents embodiment of the device, comprising the oxygenator unit integrated with a heat exchanger and blood filter and a centrifugal pump,
- Fig. 8: - presents embodiment of the device comprising a six R/NIR radiation emitters and the oxygenator module integrated with a heat exchanger, blood filter and the centrifugal pump,
- Fig. 9: - presents embodiment of the device comprising a R/NIR radiation emitter embedded to the oxygenator module,
- Fig. 10: - presents embodiment of an device comprising a fiber optic system for transmitting the R/NIR radiation to the oxygenator module case,
- Fig. 11: - presents embodiment of the device including the fiber optic system transmitting R/NIR radiation to the housing and the interior of the oxygenator module.

### Example 1

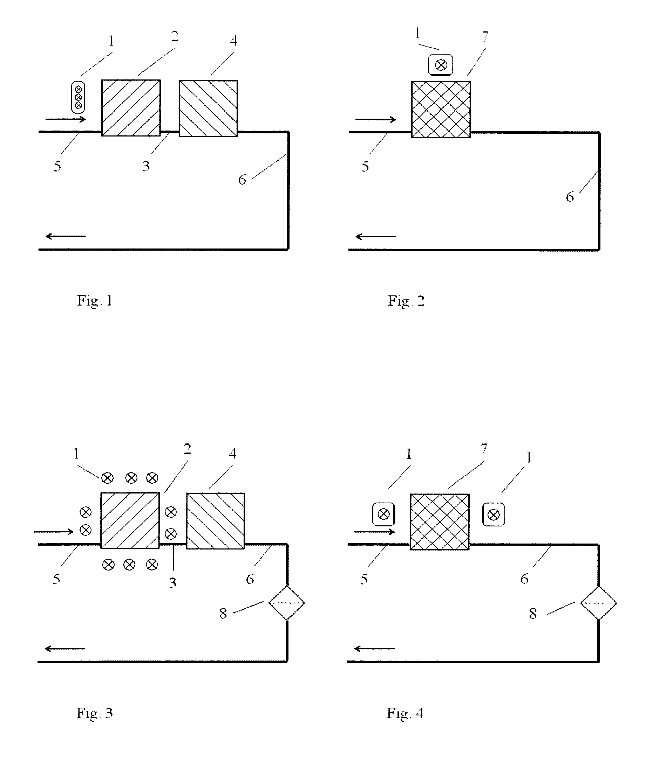

The device for photobiomodulation of blood during extracorporeal circulation, presented on Fig. 1, comprises an emitter of R/NIR radiation **1** in the form of three LEDs, the first of which produces EM waves with length of 700 nm, a second - 830 nm, and the third - 1500 nm, and the irradiance of 10 mW/cm². The emitter **1** is located next to the oxygenator **2,** connected with the drain **3** to heat exchanger **4.** Futhermore, the device includes a drain **5** suppling blood to the oxygenator **2** and drain **6,** which drains the oxygenated and thermally stable blood from the heat exchanger **4.**

In the device the patient's blood flows throught drain **5** in the direction of the arrow to the oxygenator **2,** irradiated with R/NIR radiation, by the LEDs **1,** where it is the subjected to photobiomodulation. The blood is then directed to a heat exchanger **4,** from which the irradiated, thermally stabilized and oxygenated blood is returned to patient with the drain **6.**

### Example 2

The device for blood photobiomodulation during extracorporeal circulation, illustrated by Fig. 2, is constructed as in Example 1, except that the R/NIR emitter comprises a one halogen lamp with a band-pass filter which is transparent for R/NIR wavelengths, is emitting radiation in the range of 700 - 1500 nm and the irradiance of 50 mW/cm². The emitter **1** is located over the oxygenator module with integrated heat exchanger **7** in a common housing.

The patient's blood flows to the device through drain **5** in the direction of the arrow to the oxygenator unit integrated with the heat exchanger **7,** which is exposed to the R/NIR radiation, causing the photobiomodulating effect. Then, irradiated, and thermally stabilized oxygenated blood passes through the drain **6** in the direction of the arrow towards the patient.

### Example 3

The device for blood photobiomodulation during extracorporeal circulation, presented on Fig. 3, is constructed as in Example 1, except that it comprises the R/NIR emitter **1** in the form of 10 lasers, 4 of which emit radiation with wavelength of 755 nm, 3 consecutive - radiation with a wavelength of 810 nm, 2 - radiation with a wavelength of 1064 nm, and 1 - radiation with a wavelength of 1320 nm, and the total irradiance does not exceed 10 000 mW/cm². Lasers **1** are situated around the oxygenator **2,** connected with heat exchanger **4** by the drain 3. The heat exchanger is connected with blood filter **8** with the drain **6.** In the device, the patient's blood flows through drain **5** in the direction of the arrow to the oxygenator **2,** which is exposed to the R/NIR radiation. Blood flows through the heat exchanger **4,** and then through the blood filter **8.** Irradiated, thermally stabilized and oxygenated blood passes in the direction of the arrow towards the patient.

### Example 4

The device for blood photobiomodulation during the extracorporeal circulation, shown in Fig. 4, is constructed as in Example 2, except that the R/NIR emitter **1** is the incandescent lamp combined with a band-pass filter for R/NIR wavelengths, which emits EM waves with a length of 700 - 1100 nm and a fluorescent lamp emitting EM waves with length of 700 - 850 nm. Lamps are located on both sides of the oxygenator unit integrated with heat exchanger **7,** connected to a blood filter **8.** Direct irradiation of blood with the irradiance of 5 mW/cm² in the case of the incandescent lamp, and 1.5 mW/cm² in the case of the fluorescent lamp has the photobiomodulating effect.

### Example 5

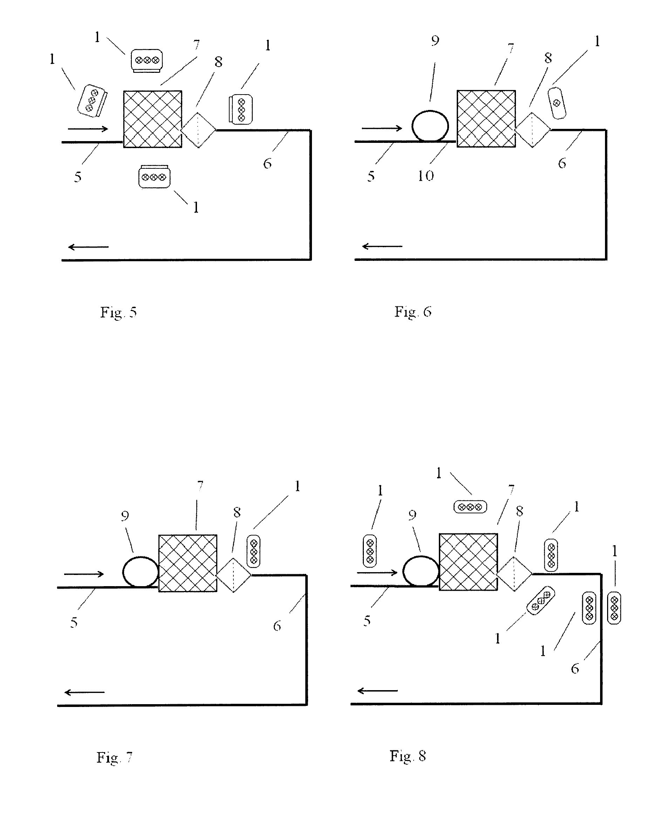

The device for the blood photobiomodulation during extracorporeal circulation, illustrated by Fig. 5 is constructed as in Example 2, except that the emitter comprises R/NIR lamps **1** in the form of LEDs emitting radiation of the visible range 370 - 500 nm, which irradiate the luminescent material, what causes the emission of radiation from the range of the R/NIR with a maximum intensity at a wavelength of 1064 nm and the irradiance of 0.5 mW/cm². Light sources are located on four sides of the oxygenator module with integrated heat exchanger **7** and the blood filter **8.**

### Example 6

The device for blood photobiomodulation during extracorporeal circulation, presented on Fig. 6, is constructed as in Example 5, except that the R/NIR emitter **1** comprises one LED, which emits EM radiation with a wavelength of 700 nm and the irradiance of 100 mW/cm², located near the oxygenator unit integrated with the heat exchanger **7** and a blood filter **8.** Moreover, device comprises a roller pump **9** positioned upstream of the oxygenator connected to it with the drain **10.**

In the device the patient's blood flows through the drain **5** to the pump **9.** which drives the blood flow through the drain **10** to the oxygenator module integrated with the heat exchanger **7** and a blood filter **8,** what results in the direct irradiation of blood with the irradiance of 0.5 mW/cm², with the photobiomodulating effect. Then, exposed, thermally stabilized and oxygenated blood flows toward the patient.

### Example 7

The device for blood photobiomodulation during the extracorporeal circulation, shown in Fig. 7, is constructed as in Example 6 except that the R/NIR emitter **1** comprises a sodium lamp with a R/NIR filter, emitting EM waves in the range of 700 - 750 nm and the irradiance of 1000 mW/cm². The emitter **1** is parallel to the oxygenator unit integrated with the heat exchanger **7,** a blood filter **8** and a centrifugal pump **9.** Irradiation of the flowing blood results in the photobiomodulating effect.

### Example 8

The device for blood photobiomodulation during extracorporeal circulation, illustrated by Fig. 8, is constructed as in Example 7, except that it includes six R/NIR emitters **1,** each of them consisting of three LEDs. Emitters **1** are located next to oxygenator module integrated with a heat exchanger **7,** and a blood filter **8** and around the drain **6.** Futhermore, two emitters emit EM radiation with the wavelength of 750 nm and the irradiance of 30 mW/cm², the next two emit EM radiation with the wavelength of 830 nm and the irradiance of 15 mW/cm², and the other two emit EM radiation with the wavelength of 950 nm and the irradiance of 10 mW/cm².

### Example 9

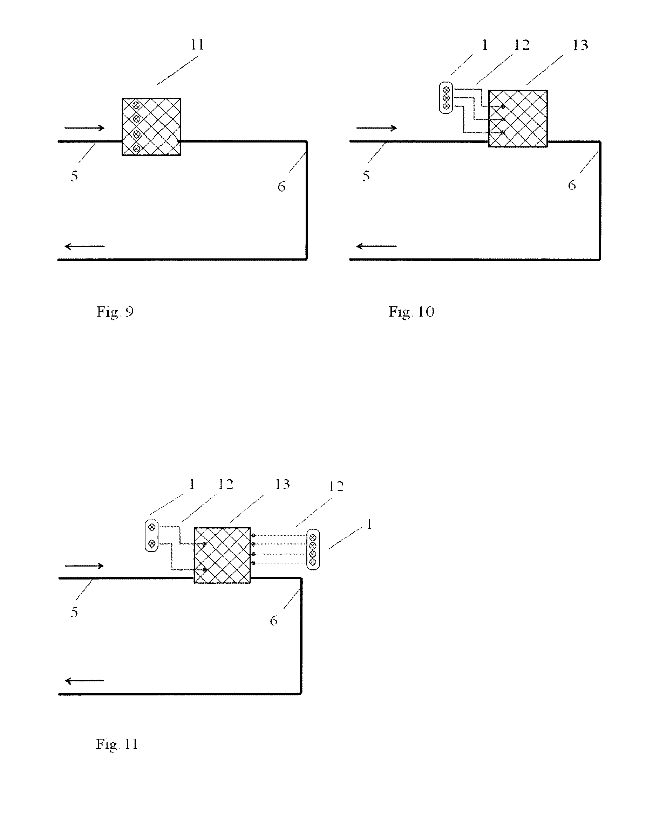

The device for blood photobiomodulation during extracorporeal circulation, presented on Fig. 9, comprises an R/NIR emitter **1** in the form of four LEDs emitting R/NIR radiation of 700 nm and the irradiance of 0.5 mW/cm², situated in the oxygenator module integrated with the heat exchanger **11.** In the device, the patient's blood flows through drain **5** to the oxygenator unit integrated with the heat exchanger and the emitter **11** in the direction of the arrow. Due to the integration of the emitter and the oxygenator in one housing, the blood is directly exposed to R/NIR radiation of LEDs, creating the photobiomodulating effect. Then, exposed, thermally stabilized and oxygenated blood flows in the direction indicated by the arrow towards the patient.

### Example 10

The device for blood photobiomodulation during the extracorporeal circulation, presented on Fig. 10, comprises an R/NIR emitter **1** in form of three lasers, whose radiation is transmitted by the optical fiber system **12,** directly irradiating blood with R/NIR radiation of 810 nm and irradiance of 150 mW/cm², to the interior of the oxygenator module integrated with heat exchanger **13.**

### Example 11

The device for blood photobiomodulation during extracorporeal circulation, shown in Fig. 11 is constructed as in Example 10, except that it includes two R/NIR emitters **1,** the first of which compromises of 2 LEDs with a wavelength of 700 nm, connected with the optical fiber system **12** to allow direct exposure of blood to R/NIR radiation in the oxygenator module integrated with the heat exchanger **13,** and the other is 4 lasers emitting a EM radiation with the wavelength of 810 nm, which is transmitted by the optical fiber system **12** to the surface of the oxygenator module integrated with heat exchanger **13,** irradiating the blood with R/NIR radiation. The irradiance of the LEDs emitter is 50 mW/cm², and for the emitter composed of lasers is 100 mW/cm².

## Claims

1. A device for blood photobiomodulation during extracorporeal circulation, comprising the radiation emitter, oxygenator, heat exchanger and blood transporting drains, **characterized in that** the device comprises at least one emitter of radiation R/NIR (1), the oxygenator (2) and a heat exchanger (4), wherein the emitter of R/NIR (1) is positioned in relation to the oxygenator (2) or heat exchanger (4) in the way enabling the irradiation of flowing blood.

2. A device according to claim 1, **characterized in that** it comprises at least one blood filter (8).

3. A device according to claim 1, **characterized in that** it comprises at least one pump (9), preferably a roller pump or centrifugal pump for assisting blood flow.

4. A device according to claim 1, **characterized in that** the oxygenator is integrated with the heat exchanger (7).

5. A device according to claim 1, **characterized in that** the oxygenator is integrated with the heat exchanger (7) and a blood filter (8).

6. A device according to claim 1, **characterized in that** the oxygenator is integrated with the heat exchanger (7), a blood filter (8) and a pump (9).

7. A device according to claim 1, **characterized in that** the emitter (1) comprises at least one EM wave source emitting radiation of one or more wavelengths in the range from 700 to 1500 nm.

8. A device according to claim 1 or 7, **characterized in that** the wave source is selected from the following group: LED, incandescent, fluorescent, sodium, halogen, laser lamp or other device that emits light of one or more wavelengths in the range from 700 to 1500 nm or emitting light of a different spectral range which can be converted to light in the range of 700 to 1500 nm.

9. A device according to claim 1, **characterized in that** the distribution of EM wave sources in the space enable to provide uniform irradiation of the flowing blood.

10. A device according to claim 1, **characterized in that** it comprises the emitters (1) irradiating the blood filter (8) or heat exchanger (4) or pump (9) assisting the blood flow or blood transporting drain.

11. A device according to claim 1 or 2 or 3 or 4 or 5 or 6, **characterized in that** the R/NIR emitters (1) are an integral part of the oxygenator (2) or heat exchanger (4) or the blood filter (8) or oxygenator module (7) or drain or a pump (9) assisting the blood flow.

12. A device according to claim 1 or 2 or 3 or 4 or 5 or 6, **characterized in that** it is provided with a fiber optic system (12), transmitting the R/NIR radiation from the emitter (1) directly into the blood flowing through the oxygenator (2) or heat exchanger (4) or a blood filter (8) or oxygenator module (7) or a pump (9) or drains transporting the blood.

13. A device according to claim 1 or 2 or 3 or 4 or 5 or 6, **characterized in that** it is provided with a fiber optic system (12) transmitting the R/NIR radiation from the emitter (1) to the surface of the oxygenator (2) or heat exchanger (4) or blood filter (8) or oxygenator module (7) or a pump (9) or drain transporting the blood.

14. A device according to claim 1, **characterized in that** the irradiance reaching the blood is in the range of 0.5 mW/cm² to 10000 mW/cm².

15. A device according to claim 1, **characterized in that** it is connected to the cardiotomy container with blood transporting drains.

16. A device according to claim 1, **characterized in that** the drains are connected to hemofilters with transporting blood drains.
